# EUROPEAN PATENT APPLICATION

(11) **EP 4 659 590 A1**
(43) Date of publication of application: **10.12.2025**
(21) Application number: 24180749.4
(22) Date of filing: 07.06.2024
(51) Int. Cl.: A23L 3/00, A23L 3/28, C02F 1/32

(54) **UV-GERMICIDAL IRRADIATION SYSTEM FOR TREATMENT OF BRINE**

(71) Applicant: Lyras DK ApS, 9000 Aalborg (DK)
(72) Inventor: STRAZNICKAS, Eimantas, 9000 Aalborg (DK); KRISTENSEN, Mathias Kræmmergaard, 9000 Aalborg (DK)
(74) Representative: Aera A/S

(57) **Abstract**

Disclosed herein is a UV-germicidal irradiation system for treatment of brine, the use of a UV-germicidal irradiation system for treatment of brine and a method for UV-germicidal irradiation treatment of brine using a UV-germicidal irradiation system comprising a UV-germicidal irradiation device and an air-cooling system.

## Description

The invention relates to the use of a UV-germicidal irradiation system for treatment of brine, to a method for UV-germicidal irradiation treatment of brine and to a UV-germicidal irradiation system for treatment of brine.

### Background

Brine is often used in the food industry, e.g. in connection with infusion of proteins with salt, sugar and flavour. Brine may for example be used for tenderizing and/or moisturizing meat. In the process for producing cheese, such as salty and/or pickled cheese, e.g. feta, domiati, halloumi, and mozzarella cheese, the cheese may be matured in brine normally in an airtight or semi-permeable container. This process gives the cheese good stability, inhibiting bacterial growth even in warmer climates.

In order to ensure a non-contaminated brine, pasteurization of the brine is often used for killing microorganisms, such as bacteria, mold, spores, protozoa or virus, in the brine. However, pasteurization may introduce problems due to the heating involved in the process. Overall, heating of the brine in terms introduces a cooling requirement. Further, heating of brine may introduce further requirement to the cleaning of the equipment, since proteins sticking to the equipment due to the heating during pasteurization are more difficult to remove. Additionally, when using pasteurization as means for killing microorganisms, an additional need for extra infrastructure providing hot water is needed.

Ultraviolet (UV) reactor instruments have previously been used for pasteurization of liquid food products. Examples of such instruments can be found in US 2002/096648, which discloses a reactor for irradiating ultraviolet light into a fluid reaction medium. An irradiation chamber is connected to an inlet and an outlet which allows the reaction medium to flow through the reactor while being exposed to ultraviolet light.

When using ultraviolet light sources in a UV-germicidal irradiation system around 30-38% of energy emitted by the ultraviolet (UV) light sources is emitted as UV light. The remaining energy is emitted as visible light and heat radiation, of which by far the largest part of the remaining energy is emitted as heat radiation. In traditional systems for UV-treatment as described in e.g. in US 2002/096648, the vast majority of the energy submitted from the light source is transferred to the fluid, and the fluid being treated will thereby normally absorb and dissipate the energy provided thereto in the form of e.g. heat. However, if the liquid is recirculated and continuously heated and/or exposed to a very energy-intensive treatment, the absorbed heat in a traditional setup result in a subsequent need to cool the liquid. This is expensive and cumbersome and often entails a risk of recontamination due to increased complexity in the additional infrastructure in the system. Certain liquids may further be temperature sensitive, where temperature changes of 10-20 degrees C may cause unwanted changes in the quality of the liquid.

An air-cooling system may be used for cooling the light sources, the electronics, and the liquid being treated in the UV-germicidal irradiation system. Air for cooling electronic equipment and the light sources is most often exchanged with the immediate surroundings. This provides for an efficient and cheap cooling of the UV-germicidal irradiation system.

However, especially in food production, there is often a high content of salt, detergents, grease or other aerosols, in the ambient air surrounding the UV-germicidal irradiation system. These factors, often combined with high humidity, may cause severe corrosion or coatings inside the UV-germicidal irradiation system, which may be destructive for the systems construction and functionality. This is in particular a situation occurring when treating brine, as brine to be treated is normally stored in a non-airtight container or even an open container positioned next to the UV-germicidal irradiation system. Vapour having a high salt concentration from the brine-containing container will therefore fill the room in which the container is positioned.

In order to cool the electronic equipment and the light sources in UV-germicidal irradiation system, while at the same time to provide protection of the metallic and electronic parts and the light sources from corrosion, the UV-germicidal irradiation system can be positioned in a different room than the one in which the brine solution is kept in. Alternatively, the brine can be kept in an airtight container thereby protecting the surroundings around the brine container. Yet alternatively, filters for removing unsuitable aerosols and particles such as the salt particles may be used for filtering the ambient air before using it for cooling the UV-germicidal irradiation system. However, it may be difficult in practice to either keep the brine container and the UV-germicidal irradiation system in separate rooms as the brine needs to be efficiently transferred between the two systems, or efficiently ensure that the brine is kept in an air-tight container, or to filter the ambient air sufficiently fine as filters in highly polluted environments often clog thereby require extensive maintenance constantly.

There is therefore a need for a new optimized solution.

### Summary

Disclosed herein in a **first aspect** is the use of a UV-germicidal irradiation system for treatment of brine, wherein the UV-germicidal irradiation system comprises a UV-germicidal irradiation device and an air-cooling system. Disclosed herein in a **second aspect** is a UV-germicidal irradiation system for treatment of brine.

The UV-germicidal irradiation device according to the **first aspect** and the **second aspect** is positioned inside a first room having ambient air surrounding the UV-germicidal irradiation device, the ambient air having an average salt-air concentration of at least 10 µg salt/m³ air.

The UV-germicidal irradiation device comprises at least one tube having:
∘ an inlet section comprising a tube inlet through which the brine enters the tube;
∘ an outlet section comprising a tube outlet through which the brine exits the tube: and
∘ a fluidic pathway extending along a longitudinal axis from the inlet section to the outlet section.

The UV-germicidal irradiation device further comprises one or more light sources configured to illuminate the fluidic pathway, wherein the one or more light sources emit light with at least one wavelength within a wavelength range between 180 nm and 315 nm.

Additionally, the UV-germicidal irradiation device comprises one or more interior compartments, wherein at least the one or more light sources are confined inside the one or more interior compartments wherein the one or more interior compartments are configured for preventing ambient air from entering into the one or more interior compartments.

The air-cooling system is configured for utilizing outside air from outside the first room for:
A. direct cooling of the one or more light sources by providing the one or more interior compartments with the outside air as cooling air, or
B. indirect cooling of the one or more light sources by the air-cooling system comprising an internal air pipe system containing internally recycled air, wherein the recycled air is provided to the one or more interior compartments as cooling air for cooling the one or more light sources, and wherein the outside air is cooled recycled air.

The outside air has a salt concentration below 5 µg salt/m³ air.

Alternatively, or in addition, the outside air has a salt concentration lower than that of the ambient air by at least 10%, such as at least 20%, such as at least 30%, such as at least 40%, such as at least 50%, such as at least 60%, such as at least 70%, such as at least 80%, such as at least 90%, such as at least 95%.

Disclosed herein in a **third aspect** is a method for UV-germicidal irradiation treatment of brine using a UV-germicidal irradiation system comprising a UV-germicidal irradiation device and an air-cooling system. The UV-germicidal irradiation system is as described in connection with the **first aspect** and the **second aspect.**

The method of the **third aspect** comprises:
- providing brine to the UV-germicidal irradiation device positioned in the first room with the ambient air having the average salt-air concentration of at least 10 µg salt/m³ air surrounding the UV-germicidal irradiation device;
- performing UV-germicidal irradiation treatment of the brine in the UV-germicidal irradiation device;
- providing outside air from outside the first room to the air-cooling system; and
- cooling the one or more light sources inside the one or more interior compartments directly or indirectly with the outside air by means of the air-cooling system.

The outside air from outside the first room has a salt concentration below 5 µg salt/m³ air.

Alternatively, or in addition, the outside air has a salt concentration lower than that of the ambient air by at least 10%, such as at least 20%, such as at least 30%, such as at least 40%, such as at least 50%, such as at least 60%, such as at least 70%, such as at least 80%, such as at least 90%, such as at least 95%.

Direct cooling of the one or more light sources is obtained by providing the outside air as cooling air to the one or more interior compartments. Indirect cooling of the one or more light sources is obtained by providing the one or more interior compartments with recycled air as cooling air for cooling the one or more light sources, and cooling the recycled air with the outside air.

Performing UV-germicidal irradiation treatment of the brine in the UV-germicidal irradiation device and cooling the one or more light sources inside the one or more interior compartments may be done simultaneously.

By brine is understood a water-based solution with a high concentration of salt. Examples of salt used in a brine is sodium chloride, calcium chloride, bromides, and formates. Brine may refer to a water-based solution with salt solutions ranging from about 3.5 % w/w salt up to about 26% w/w, such as salt solutions in the range of 8% w/w - 25 % w/w salt. The temperature when measuring these concentrations are normally around room temperature. Alternatively, the temperature may vary between 8 degrees Celsius and 23 degrees Celsius.

By average salt-air concentration of X µg salt/m³ air is meant that in a volume of 1 m³, the salt content in the air is X µg on average. Examples of salt contents found in the air in a room with brine are sodium chloride, calcium chloride, bromides, and formates. By salt concentration of at least 10 µg salt/m³ air is thereby meant the combined salt concentration is at least 10 µg salt/m³ air, where the combined salt concentration possibly contains more than one type of salt. The salt concentration will also include any minor salt components, which are normally found in air in general.

By outside air is meant air being different from the air inside the first room. This may be atmospheric air from outside the building in which the UV-germicidal irradiation device is positioned. Normally, outside air contains substantially less salt compared to the air inside the first room. By outside air may also be meant air, which is substantially isolated from the air inside the first room to the extent that mixing between the two volumes of air do not occur at a level affecting key quality parameters of either.

By the use of a UV-germicidal irradiation system for treatment of brine as described above, where outside air is used directly or indirectly for cooling the UV-germicidal irradiation device is obtained an improved solution, which both ensures an efficient cooling of the light sources, and the stability of the electronic parts in the UV-germicidal irradiation device, while at the same time reduces or even avoids the problems with corrosion. The use of a UV-germicidal irradiation system is further a financially very attractive alternative to other solutions including:
∘ transferring the brine solution between different rooms,
∘ shielding the brine containing container completely to avoid the high salt content in the ambient air around the UV-germicidal irradiation device, and/or
∘ using high-quality, expensive and maintenance-intensive filters for removing undesired aerosols and particles such as the salt particles from the ambient air.

The simplicity of using air from outside the first room provides a cheap solution. The outside air may be provided to the UV-germicidal irradiation device for cooling the light sources directly or indirectly. However, the system is a closed system in the sense that ambient air with a high salt concentration is prevented from entering the system.

Using UV-light for treatment of brine is also referred to as using cold pasteurization for treatment of brine. One of the advantages of using light radiation as a means for cold pasteurization is that it is a very energy-efficient way for partial sterilization. Pasteurization is not only limited to partial sterilization of a substance and especially a liquid at a temperature and for a time period of exposure that destroys objectionable organisms without major chemical alteration of the substance, but also covers cold pasteurization which is partial sterilization of a substance and especially a liquid in a process where heat is evaded as the main eradication of objectionable organisms without major chemical alteration of the substance. With evaded is not meant excluded but reduced. The fluidic pathway is normally designed to provide a high surface to volume ratio, increasing the exposure of light energy per unit volume with reduced self-shadowing effects from the brine being treated. In this manner it is possible to treat brine using light when the material, creating the fluidic pathway, is at least partly transparent to the radiation of light. The fluidic pathway is normally designed to provide a high surface to volume ratio, increasing the exposure of light energy per unit volume with reduced self-shadowing effects from the brine being treated. In this manner it is possible to treat brine using light when the material, creating the fluidic pathway is at least partly transparent to the radiation of light.

In one or more examples, the one or more interior compartments has an interior compartment air pressure being higher than an ambient air pressure of the ambient air in the first room thereby preventing ambient air from entering into the one or more interior compartments. By making the air pressure in the interior compartment higher than that of the ambient air, the ambient air is prevented from entering into the one or more interior compartments. The one or more interior compartments may also/alternatively form a substantially airtight system.

In one or more examples, the air-cooling system comprises one or more fans configured for transferring the cooling air into the UV-germicidal irradiation device. The one or more fans may be configured for rotating at an adjustable velocity.

If the temperature of the outside air is lower than normal, the outside air more efficiently cools the light sources either directly or indirectly. The adjustable velocity may therefore be decreased to obtain the same cooling efficiency as with a higher temperature of the outside air. This ensures a long lifetime of the light sources if these are dependent on a uniform cooling independent on the temperature of the outside air.

In general, an increase in the adjustable velocity will introduce an increase in the cooling of the one or more light sources. Likewise, a decrease in the adjustable velocity will introduce a decrease in the cooling of the one or more light sources. The temperature regulation of the outside air is not a requirement, but may be an advantage if the temperature of the outside air vary depending on the temperature in the area from which the outside air is taken from. This is particularly true if the outside air is taken from outside the building in which the UV-germicidal irradiation system is positioned when the building is in a climate, where large temperature changes occur and/or where there is a large difference between day and night temperatures.

The UV-germicidal irradiation device will normally experience an increase of the temperature inside the interior compartments during use as the different parts in the device are heated. The requirements to the temperature of the outside air may therefore change during use of the UV-germicidal irradiation system.

It may further be needed to lower / increase the light source output during use. It may also be used for saving energy or for protecting the brine solution and preventing overexposure of the same. When reducing the light source output, a lower amount of cooling is often needed.

In one or more examples, the one or more light sources comprises an adjustable light source output. Changing the light source output may optimize the UV-treatment. An interplay between an adjustment of the light source output and the outside air temperature is often used for facilitating optimal conditions of UV-germicidal irradiation device.

In one or more examples, the UV-germicidal irradiation system comprises a temperature sensor for measuring a temperature of the cooling air entering into the UV-germicidal irradiation device. The measurement of the temperature of the cooling air may be used together with the light source output to constantly adjust the velocity of the fans to provide an optimized temperature inside the UV-germicidal irradiation device.

In one or more examples, the UV-germicidal irradiation device further comprises one or more perforated plates defining the one or more interior compartments. The perforated plates may be used for assisting in ensuring a positive pressure inside UV-germicidal irradiation device and to assist in directing the airflow through and between the perforated plates and out of the UV-germicidal irradiation device.

In one or more examples, the one or more perforated plates are configured for ensuring that the interior compartment air pressure is higher than an ambient air pressure of the ambient air in the first room.

In one or more examples, the UV-germicidal irradiation device comprises a second interior compartment with a second interior compartment air pressure, the second interior compartment being positioned between the one or more interior compartments containing the one or more light sources and the air-cooling system, wherein the second interior compartment air pressure is higher than the first mentioned interior compartment air pressure. The outside air is thereby led efficiently through the interior compartments.

In one or more examples, the air-cooling system comprises an inlet air pipe with an inlet pipe opening, the inlet pipe opening being configured for being positioned outside the first room. The inlet air pipe may be a traditional air shaft.

In one or more examples, the air-cooling system comprises one or more coarse filters configured for filtering larger particles from the cooling air before the cooling air enters into the UV-germicidal irradiation device. The one or more coarse filters may be positioned along the inlet air pipe. Thus, before entering the air-cooling system, the outside air may pass a coarse filter.

In one or more examples, the air-cooling system comprises a de-humidifier configured for lowering the humidity of cooling air before the cooling air enters into the UV-germicidal irradiation device. The de-humidifier may be positioned along the inlet air pipe. Thus, before entering the air-cooling system, the outside air may pass a de-humidifier.

In one or more examples, the UV-germicidal irradiation device comprises an outlet opening through which the outside air exits the UV-germicidal irradiation device as exhaust air. The exhaust air may exit through the outlet opening and into the first room. Alternatively, the UV-germicidal irradiation device may comprise an outlet air pipe with the outlet opening, wherein the outlet air pipe extends to a position outside the first room, whereby the exhaust air is led to a position outside the first room.

In one or more examples, the air-cooling system comprises one or more particle filters configured for filtering fine particles from the outside air and/or internally recycled air before the outside air and/or internally recycled air enters into the UV-germicidal irradiation device. This is seen as an additional add-on to the system.

In one or more examples, the system further comprises a temperature regulating system comprising a temperature sensor configured for measuring a temperature of the outside air, and/or the recycled air, and/or the cooling air entering into the air-cooling system, and an air regulating system, wherein the air regulating system is configured for 1) heating the cooling air entering the one or more interior compartments if the temperature of the outside air, and/or the recycled air, and/or the cooling air measured by the temperature sensor is below a first pre-set temperature value, and/or 2) cooling the air entering the one or more interior compartments if the temperature of the outside air, and/or the recycled air, and/or the cooling air measured by the temperature sensor is above a second pre-set temperature value. By a temperature regulating system is obtained an improved control of the brine being treated as the cooling system and the temperature of the cooling air is controlled to a high degree. This may be relevant in environments where the outside air temperature varies due to e.g. seasonal climate changes and the like.

The temperature regulating system may be an air-to-liquid heat exchange system. Thus, in one or more examples, the air-cooling system is connected to an air-to-liquid heat exchange system, the air-to-liquid heat exchange system comprising one or more cooling liquid containers containing a cooling liquid, wherein the air-to-liquid heat exchange system is configured for heating or cooling the outside air before the outside air enters into the UV-germicidal irradiation device. The air-to-liquid heat exchange system may be positioned along the inlet air pipe. As an alternative to the air-to-liquid heat exchange, an electrical temperature regulation system may be used. Both types of temperature regulation systems are advantageous for regulating the temperature of the outside air in a climate, where large temperature changes occur and/or where there is a large difference between day and night temperatures and/or large differences in humidity occurs. A larger stability of the UV-germicidal irradiation system may thereby be obtained. Also, regulating the temperature of the outside air before it enters into the air-cooling system may also form an interplay with the fans in the air-cooling system, thereby reducing the need to vary the adjustable velocity.

In one or more examples, exhaust air and/or recycled air is led though the air-to-liquid heat exchange system thereby heating the incoming outside air. The exchange of heat allows for a cooling of the exhaust air and/or recycled air before entering into the one or more interior compartments again.

In one or more examples, the UV-germicidal irradiation system comprises a top flank and wherein the air-cooling system is positioned as part of the top flank. The UV-germicidal irradiation system may further comprise additional flanks including a bottom flank, a first side flank, a second side flank, a third side flank, and a fourth side flank.

In one or more examples, the fluidic pathway is a coil section in the form of a spiral-shaped tube. The fluidic pathway of the one or more tubes may utilize the flow regime occurring when the media is traveling in a spiral-shaped tube creating the fluidic pathway. The flow regime in the fluidic pathway may consist of one or several eddies, which creates a secondary flow axial on the primary flow utilizing the centrifugal force (e.g. Dean vortex flow) to enhance the fraction of liquid being transported to, and thus exposed to UV light at, the tube surface being exposed to UV-light emitted by the light sources. The fluid movement through the fluidic pathway may have a double vortexual pattern consistent with a Dean vortex flow. This provides an axial flow in the fluidic pathway, ensuring that a high volume gets in contact with the surface. This may decrease the distribution of the exposure of light energy per unit volume/surface area with reduced self-shadowing effects from the brine being treated. Thus, in one or more examples, a fluid movement through the fluidic pathway creates a Dean Vortex flow, laminar flow, or turbulent flow in the liquid. This is in particular obtainable using a coiled spiral shaped fluidic pathway. An advantage of utilizing a Dean Vortex, laminar, or turbulent flow, in a double helix pattern, is that it may increase the volume getting in contact with the surface and thereby the exposure of light energy with reduced self-shadowing effects from the brine being treated. Thus, less energy is required for treatment of the liquid.

In one or more examples, the UV-germicidal irradiation device further comprises at least one elongated support structure, wherein the spiral-shaped tube is coiled around the elongated support structure along the longitudinal axis of the fluidic pathway. An advantage using a support structure to coil the spiral-shaped tube around is that a support structure stabilizes the spiral-shaped tube, if said tube is e.g. made of a flexible material. The support structure may hence provide stabilization for the coil. Additionally, the support structure may have other advantage, e.g. helping with enhancing the amount of light radiated to the spiral-shaped tube by being e.g. reflective. The elongated support structures may include a solid support structure, a cylindrical mesh or anything in between. Thus, in one or more examples, the plurality of support structures is made of or covered by a reflective material, such as a reflective polymeric material or a polished metal.

In one or more examples, the UV-germicidal irradiation device comprises one or more light source filters positioned between the one or more light sources and the fluidic pathway of the at least one tube, wherein the one or more light source filters are configured for preventing light above a wavelength of 315 nm from reaching the fluidic pathway of the at least one tube. By "prevent light above a wavelength of 315 nm from reaching the fluidic pathway of the one or more tubes" is meant that the one or more filters attenuates light above 315 nm by at least a factor 10, such as by at least a factor 100, such as by at least a factor 1.000, such by at least a factor 10.000. One of the advantages of using one or more filters is that photo oxidation from higher wavelengths may be avoided. Additionally, the filters may avoid hot air from contacting the fluidic pathway of the tube, hereby avoiding heating of the brine.

In one or more examples, the UV-germicidal irradiation device comprises a plurality of tubes arranged in one or more tube sections, and a plurality of light sources arranged in one or more light source sections, wherein the tube sections and the light source sections are alternatingly arranged in a horizontal direction or a vertical direction.

The light source sections may be separated from the tube sections. The separation of the light source sections and the tube sections may potentially prevent leaks due to spray/distribute by the cooling air flow from the cooling system.

The structure separating the light source sections and the tube sections may incorporate and/or encompasses areas of UV transparent materials at positions that allow a significant amount of the UV radiation from the light sources to pass from the light source sections into the tube sections. Thus, in one or more examples, the light source sections are separated from the tube sections by one or more UV transparent materials. The material may also be partly UV transparent such that the transparency varies over the material. The separation into different sections by a UV transparent material allows for a separation of the system into different thermal zones depending on the section type. As the light sources will normally submit heat to the surroundings during use, the sections with the light sources will normally be heated during use to a higher temperature than the sections with the tubes. In one or more examples, the light sources operate at a light source temperature between 0 °C and 120 °C, such as between 20 °C and 60 °C, such as between 30 °C and 50 °C.

In one or more examples, each light source cassette also comprises one or more of the one or more filters prevent light above a wavelength of 315 nm from reaching the fluidic pathway of the one or more tubes.

In one or more examples, the at least one tube is made of a polymeric or quartz glass material being at least ultraviolet light transparent at wavelengths between 250 and 260 nm, such as between 240 and 270 nm, such as between 230 and 280 nm, such as between 210 and 290 nm, such as between 195 and 305 nm, such as between 180 and 315 nm. By a polymeric material being at least ultraviolet light transparent is meant that at least 5% of UV-light at the given wavelengths are allowed to pass through 0.1 mm of said material. In one or more examples, the polymeric material has an ultraviolet transmittance (UVT) of at least 5%, such as at least 10%, such as at least 15% at wavelengths between 250 and 260 nm, wherein the UVT is measured with a 0.1 mm path length.

In one or more examples, the at least one tube is made from a polymeric material selected from amorphous fluoropolymer (AF), polypropylene (PP), fluorinated ethylene propylene (FEP), polyethylene (PE), polytetrafluoroethylene (PTFE), perfluoroalkoxy alkanes (PFA), polyvinylidene fluoride (PVDF), nylon, rubber, latex, acrylic glass, polycarbonate (PC), polystyrene, acrylonitrile butadiene styrene (ABS), a quartz material, or a similar material and/or combinations thereof.

In one or more examples, the at least one tube is made from amorphous fluoropolymer (AF). Amorphous fluoropolymer (AF) is a family of amorphous fluoroplastics. These materials are similar to other amorphous polymers in optical clarity and mechanical properties, including strength. These materials are comparable to other fluoroplastics in their performance over a wide range of temperatures, in having excellent chemical resistance, and in having outstanding electrical properties. AF polymers are distinct from other fluoroplastics in that they are soluble in selected solvents, have high gas permeability, high compressibility, high creep resistance, and low thermal conductivity. AF polymers have the lowest dielectric constant of any known solid polymer. AF polymers have a low index of refraction when compared to many other known polymers.

In one or more examples, the one or more light sources are selected from a mercury-vapor light source, a xenon light source, a light emitting diode (LED), a pulsed LED, or a low pressure germicidal light source, such as a low-pressure mercury-vapor light source. A mercury-vapor light source is a gas discharge light source that uses an electric arc through vaporized mercury to produce light. The arc discharge may be confined to a small fused quartz arc tube. A xenon arc light source is a specialized type of gas discharge light source, an electric light that produces light by passing electricity through ionized xenon gas at high pressure. It produces a bright white light that closely mimics natural sunlight. A special kind of xenon light source is used in automobiles. These are actually metal-halide light sources, where a xenon arc is only used during start-up. A light emitting diode (LED) is a two-lead semiconductor light source. It is a p-n junction diode that emits light when activated. When a suitable voltage is applied to the leads, electrons are able to recombine with electron holes within the device, releasing energy in the form of photons. This effect is called electroluminescence, and the color of the light (corresponding to the energy of the photon) is determined by the energy band gap of the semiconductor. LEDs are typically small (less than 1 mm) and integrated optical components may be used to shape the radiation pattern. In one or more examples, the light sources are a metal-halide light source. A metal-halide light source is an electrical light source that produces light by an electric arc through a gaseous mixture of vaporized mercury and metal halides. It is a type of high-intensity gas discharge light source. They are similar to mercury-vapor light sources, but contain additional metal halide compounds in the quartz arc tube, which may improve the efficiency and color rendition of the light.

In one or more examples, the one or more filters are selected from bandpass filters, notch filters, or a combination of both.

In one or more examples, the UV-germicidal irradiation system is configured for inactivating or reducing a biological contaminant by an order of at least 1-Log₁₀, such as at least 2-Log₁₀, such as at least 3-Log₁₀, such as at least 4-Log₁₀, such as at least 5-Log₁₀, such as at least 6-Log₁₀.

Cheese (both yellow and white types of cheese) is sometimes left in brine pools for long periods of time. When some cheese is taken out of the pool, new cheese is added, which may introduce contamination into the brine. Contamination may also come from the surroundings as the brine pool is open to the ambient air. An additional filtration step may therefore be coupled to the UV-germicidal irradiation system. Such additional filtrating may be a kidney loop setup with a pasteurizer, a microfiltration system or a UV system. Re-looping into the same UV-germicidal irradiation system is also an option. By using an additional filtration step, the contamination may be decreased and maintained below a predefined limit. As cheese is normally added in low frequencies and the brine pools are often rather large, the rate of contamination (CFU/mL per time) is low relative to other types of processes such as "regular" pasteurization. Consequently, the log reduction through the UV-germicidal irradiation system may be relatively low, but still have high relevance. Often, cheese-producers are willing to except that it may take 3-4 weeks or even months to bring down the contamination in a brine pool in the beginning of a UV-germicidal irradiation treatment process as the UV-germicidal irradiation system described herein will allow the cheese-producers to replace more maintenance-heavy systems.

Dimensioning the UV-germicidal irradiation system may include selecting an optimum size of the spiral-shaped tube, and/or adjusting a flow regime of the liquid in the coiled fluidic pathway inside the spiral-shaped tube, and/or adjusting a light source intensity. The liquid food product flows through the spiral-shaped tube with a flow rate. In one or more examples, the flow rate measured in millilitres per minutes is between 200-80.000 ml/min. The flow rate depends on the total length of the spiral-shaped tube and the pitch of the spiral-shaped tube, i.e. how may spiral turns the tube makes within a given area. Therefore, the flow rate measured in millilitres per minutes may be between 200-80.000 ml/min, or between 500-70.000 ml/min, or between 500-56.000 ml/min, or between 500-35.000 ml/min, or between 500-23.000 ml/min, or between 750-23.000 ml/min, or between 1.000-23.000 ml/min, or between 1.200-23.000 ml/min, or between 200-6.000 ml/min, or between 500-4.000 ml/min, or between 800-2.000 ml/min, or between 900-1.100 ml/min. The predefined flow speed may be a flow speed between 1 m/s and 15 m/s, such as between 1 m/s and 10 m/s, such as between 3 m/s and 8 m/s, such as between 5 m/s and 7 m/s. Flow velocity may be defined as the mass flow divided by fluid density and internal cross-sectional area of the coil. Thus, the flow velocity is the vector field that is used to describe fluid motion in a mathematical manner. The entire length of the flow velocity is referred to as the flow speed. Flow velocity in fluids is the vector field that provides the velocity of fluids at a certain time and position. Flow velocity is also known as macroscopic velocity.

The one or more light sources may have a predefined power-output. The predefined power-output may be a power-output of the intensity of the light sources between 100 and 1200 W/m² measured at 254 nm.

### Brief description of the drawings

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example needs not have all the aspects or advantages shown. An aspect or an advantage described in conjunction with a particular example is not necessarily limited to that example and can be practiced in any other examples even if not so illustrated, or if not so explicitly described.
Figure 1 illustrates a UV-germicidal irradiation system for treatment of brine.
Figures 2A-B illustrate examples of tubes and light sources used in the UV-germicidal irradiation device of figure 1.
Figures 3A-B illustrate examples of tubes and light sources used in the UV-germicidal irradiation device of figure 1.
Figures 4A-D illustrates examples of the UV-germicidal irradiation systems positioned inside a first room.

### Description of examples

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

In the drawings, thicknesses of a plurality of layers and areas are illustrated in an enlarged manner for clarity and ease of description thereof. When a layer, area, element, or plate is referred to as being "on" another layer, area, element, or plate, it may be directly on the other layer, area, element, or plate, or intervening layers, areas, elements, or plates may be present therebetween. Conversely, when a layer, area, element, or plate is referred to as being "directly on" another layer, area, element, or plate, there are no intervening layers, areas, elements, or plates therebetween. Further when a layer, area, element, or plate is referred to as being "below" another layer, area, element, or plate, it may be directly below the other layer, area, element, or plate, or intervening layers, areas, elements, or plates may be present therebetween. Conversely, when a layer, area, element, or plate is referred to as being "directly below" another layer, area, element, or plate, there are no intervening layers, areas, elements, or plates therebetween.

The spatially relative terms "lower" or "bottom" and "upper" or "top", "below", "beneath", "less", "above", and the like, may be used herein for ease of description to describe the relationship between one element or component and another element or component as illustrated in the drawings. It will be understood that the spatially relative terms are intended to encompass different orientations of the device in use or operation, in addition to the orientation depicted in the drawings. For example, in the case where a device illustrated in the drawings is turned over, elements described as being on the "lower" side of other elements, or "below" or "beneath" another element would then be oriented on "upper" sides of the other elements, or "above" another element. Accordingly, the illustrative term "below" or "beneath" may include both the "lower" and "upper" orientation positions, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below, and thus the spatially relative terms may be interpreted differently depending on the orientations described.

Throughout the specification, when an element is referred to as being "connected" to another element, the element is "directly connected" to the other element, or "electrically connected" to the other element with one or more intervening elements interposed therebetween.

The terminology used herein is for the purpose of describing particular examples only and is not intended to be limiting. As used herein, the singular forms "a," "an," and "the" are intended to include the plural forms, including "at least one," unless the content clearly indicates otherwise. "At least one" is not to be construed as limiting "a" or "an." It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

It will be understood that, although the terms "first," "second," "third," and the like may be used herein to describe various elements, these elements should not be limited by these terms. These terms are only used to distinguish one element from another element. Thus, "a first element" discussed below could be termed "a second element" or "a third element," and "a second element" and "a third element" may be termed likewise without departing from the teachings herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

Exemplary examples are described herein with reference to cross section illustrations that are schematic illustrations of idealized examples, wherein like reference numerals refer to like elements throughout the specification. As such, variations from the shapes of the illustrations as a result, for example, of manufacturing techniques and/or tolerances, are to be expected. Thus, examples described herein should not be construed as limited to the particular shapes of regions as illustrated herein but are to include deviations in shapes that result, for example, from manufacturing. For example, a region illustrated or described as flat may have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims. Some of the parts which are not associated with the description may not be provided in order to specifically describe exemplary examples of the present disclosure.

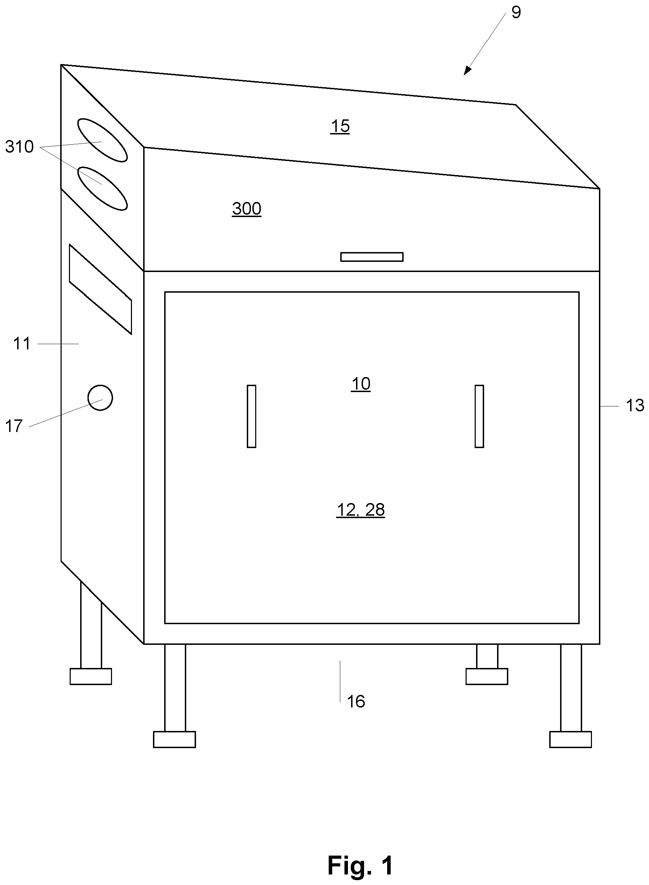

Figure 1 shows a UV-germicidal irradiation system 9 for treatment of brine. The UV-germicidal irradiation system 9 comprises a number of side flanks, including a first side flank 11, a second side flank 12, a third side flank 13, a fourth side flank 14, a top flank 15 and a bottom flank 16. On one of the side flanks is normally found connecting means for providing brine to the UV-germicidal irradiation device 10. In figure 1, this brine receiving opening 17 is illustrated on the first side flank 11 as a simple circle. On one of the side flanks is normally found door panels 28 for providing access to the inside of the UV-germicidal irradiation device 10. In figure 1, a door panel 28 is illustrated on the second side flank 12 as a simple square.

The UV-germicidal irradiation system 9 has an air-cooling system 300. The air-cooling system 300 is configured for cooling the light sources 200. In the illustration in figure 1, the air-cooling system 300 is found as constituting the top flange 15 of the UV-germicidal irradiation system 9. Other positions may also be used.

The air-cooling system 300 comprises one or more openings 310 for providing air into the air-cooling system 300 and/or allowing air from the air-cooling system 300 to exit the air-cooling system 300. In figure 1, two openings 310 are illustrated.

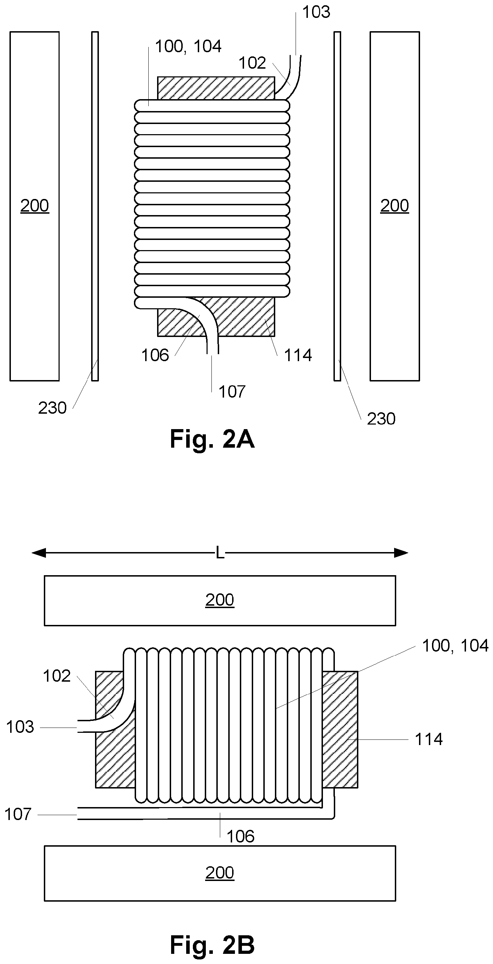

Inside the UV-germicidal irradiation UV-germicidal irradiation device 10 are found at least one tube 100 as illustrated in figures 2A and 2B. The tube 100 comprises an inlet section 102 with a tube inlet 103 through which the brine enters the tube 100. The tube 100 further comprises an outlet section 106 with a tube outlet 107 through which the brine exits the tube 100. Between the inlet section 102 and the outlet section 106 is a fluidic pathway 104 extending along a longitudinal axis L. In figure 2A, the fluidic pathway 104 is illustrated as extending along a vertical plane. In figure 2B, the fluidic pathway 104 is illustrated as extending along a horizontal plane.

The tubes 100 in figures 2A-B are shown in the form of a spiral-shaped tube. However, straight tubes or tubes having alternative shapes could also be imagined.

The tubes 100 in figures 2A-B are wound around a support structure 114 to provide stability to the tube 100. The support structure 114 may be omitted if e.g. the tube material is sufficiently rigid to maintain its position.

The UV-germicidal irradiation device 10 also comprises one or more light sources 200 configured to illuminate the fluidic pathway 104. In figures 2A-B, a light source 200 is positioned on each side of the tube 100. The light sources 200 emit light with at least one wavelength within a wavelength range between 180 nm and 315 nm.

Between the light sources 200 and the tube 100 in figure 2A is found a light source filter 230. The light source filters 230 are configured for preventing light above a wavelength of 315 nm from reaching the fluidic pathway 104. The filter 230 is not mandatory.

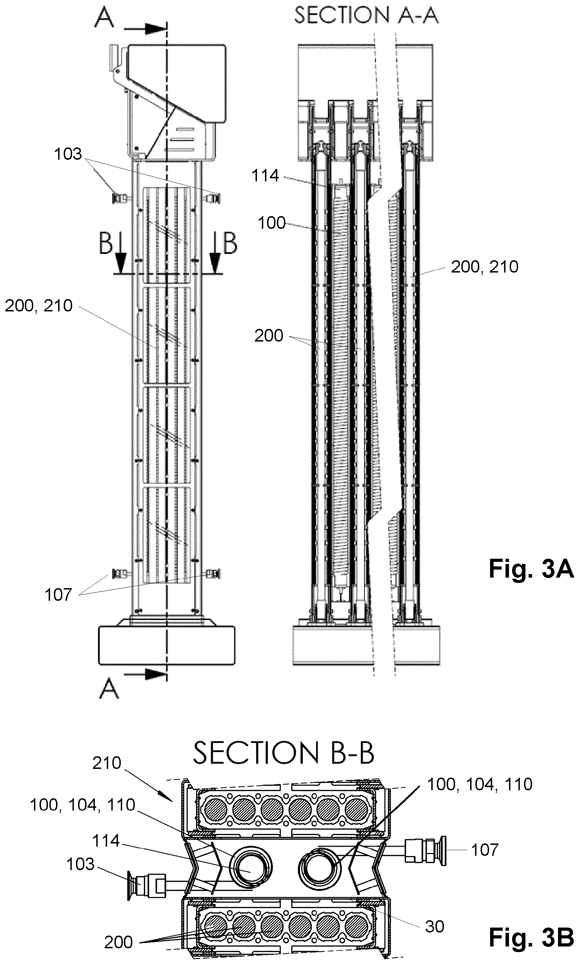

As illustrated in figures 3A-B, the light sources 200 may be arranged in light source sections 210, where each light source section 210 includes a number of light sources 200. An advantage of using light source sections 210 is that multiple light sources 200 may be removably arranged in the UV-germicidal irradiation device 10. The light source sections 210 may be positioned such that they are removable through one of the side flanks. The light source sections 210 may have an opening for allowing cooling air to pass through.

Likewise, a number of tubes 100 may be arranged in tube sections 110. The tube sections 110 may be removably arranged in the UV-germicidal irradiation device 10. The tube sections 110 may be positioned such that they are removable through one of the side flanks, possibly the same as the light source sections 210.

As illustrated in figures 3A-B, the tube section 110 and the light source sections 210 are alternatingly arranged in a vertical direction. If the tubes 100 and the light sources 200 extend along a horizontal direction, the tube section 110 and the light source sections 210 can be alternatingly arranged in the horizontal direction. The light source sections 210 may be separated from the tube sections 110 by one or more UV transparent materials 30 as illustrated in figure 3B.

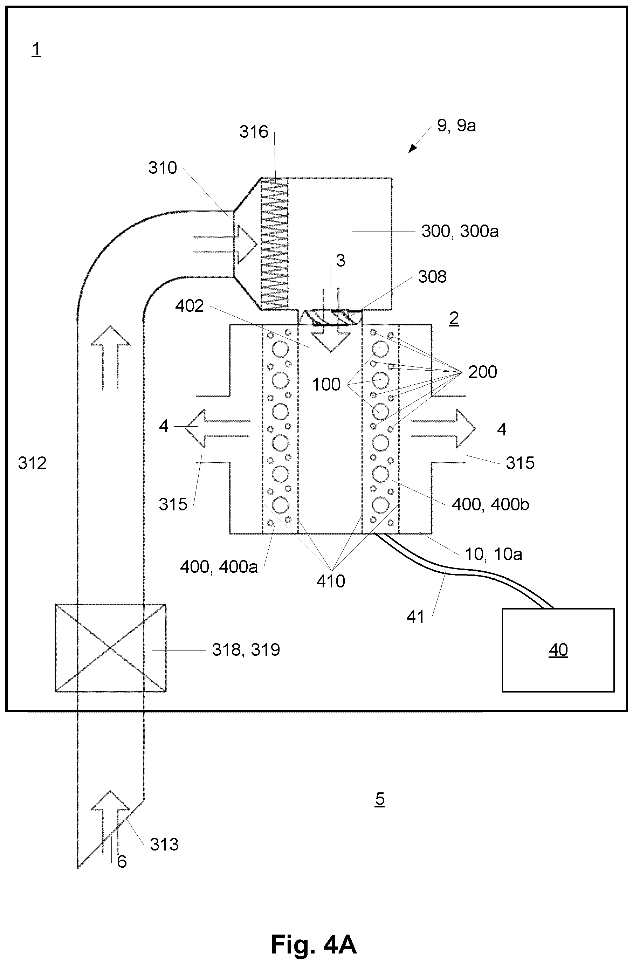

The light sources 200 inside the UV-germicidal irradiation device 10 comprises are positioned inside one or more interior compartments 400. The interior compartments 400 may be defined by plates 410, e.g. perforated plates, as illustrated in figures 4A-D.

As illustrated in figures 4A-D, the UV-germicidal irradiation system 9 is positioned inside a first room 1. The air inside the first room 1 and surrounding the UV-germicidal irradiation device 10 is referred to as ambient air 2. When treating brine in a UV-germicidal irradiation device 10 as shown in figures 4A-D, the brine solution is often kept in one or more open containers 40 positioned inside the first room 1 as shown in figure 4A, where a brine container 40 is positioned randomly in the first room 1 for illustrative purposes. Brine is typically transferred from the one or more open containers 40 to the UV-germicidal irradiation system 10 by means of tubes/pipes 41.

The very high salt content in brine will unavoidably mix with the ambient air 2 resulting in the ambient air 2 normally having an average salt-air concentration of at least 10 µg salt/m³ air. Thus, the ambient air 2 provides for a corrosive environment for any UV-germicidal irradiation devices positioned in the same room as the brine container due to the very high salt concentration of at least 10 µg salt/m³ air. This is problematic for the metallic and/or electronic parts, in particular inside the UV-germicidal irradiation device 10 as they will rapidly corrode if exposed to ambient air 2, but an inside position will make it more difficult to exchange the parts.

The air-cooling system 300 provides outside air 6 from outside 5 the first room 1 to the UV-germicidal irradiation device 10, for directly or indirectly cooling of the interior compartments 400 with the light sources 200. The outside air 6 normally has a salt concentration below 5 µg salt/m³ air and could e.g. be atmospheric air taken from outside the building in which the UV-germicidal irradiation device 10 is positioned in the first room 1. Alternatively, or in addition, the outside air 6 has a salt concentration lower than that of the ambient air 2 by at least 10%, such as at least 20%, such as at least 30%, such as at least 40%, such as at least 50%, such as at least 60%, such as at least 70%, such as at least 80%, such as at least 90%, such as at least 95%.

By using the air-cooling system 300, ambient air 2 is prevented from entering the interior compartments 400 with the light sources 200 and the tubes 100. This is illustrated in figures 4A-D. Figures 4A-D illustrates different configurations for cooling the interior compartments 400 directly or indirectly with outside air 6 using the air-cooling system 300 possibly including different add-on parts.

In figure 4A, a first UV-germicidal irradiation system 9a comprising a first UV-germicidal irradiation device 10a and a first air-cooling system 300a is shown. The first UV-germicidal irradiation device 10a comprises two interior compartments 400a, 400b each with a plurality of tubes 100 surrounded by light sources 200. The interior compartments 400a, 400b are defined by a number of perforated plates 410. The first UV-germicidal irradiation device 10a shown in figure 4A comprises a second interior compartment 402 positioned between the two interior compartments 400a, 400b. The first air-cooling system 300a uses direct cooling of the interior compartments 400 using outside air 6 as cooling air 3. Outside air 6 enters the first UV-germicidal irradiation device 10a through an inlet air pipe 312 connected to the first air-cooling system 300a. The outside air 6 enters into the inlet air pipe 312 through an inlet pipe opening 313 positioned outside the first room 1. Before entering the first air-cooling system 300a, the outside air 6 may pass an optional coarse filter 319 or an optional de-humidifier 318. The first air-cooling system 300a may have an optional particle filter 316 for filtering fine particles from the outside air 6.

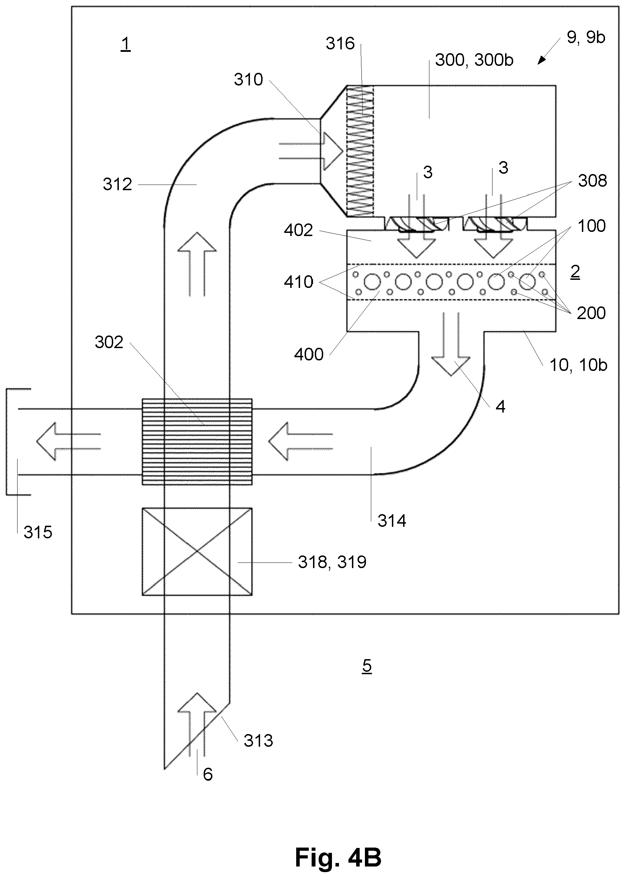

In first UV-germicidal irradiation device 10a, the outside air 6 enters into the second interior compartment 402 from where it is distributed to the interior compartments 400a, 400b before exhaust air 4 exits the first UV-germicidal irradiation device 10a through an outlet opening 315. The exhaust air 4 is directed into the first room 1, but could also be led outside the first room 5 by means of an outlet air pipe connected to the outlet opening 315. This is e.g. shown in the example shown in figure 4B.

By making the air pressure in the second interior compartment 402 higher than that in the first air-cooling system 300a, the outside air 6 flows from the the first air-cooling system 300a and into the first UV-germicidal irradiation device 10a as cooling air 3. One or more fans 308 may additionally be utilized for providing the cooling air 3 to the first UV-germicidal irradiation device 10a.

By making the air pressure in the second interior compartment 402 higher than that in the interior compartments 400a, 400b, which again is higher than that in the first room 1, the cooling air 3 flows from the second interior compartment 402 through the first UV-germicidal irradiation device 10a and out into the first room 1 as exhaust air 4. The pressure difference also ensures that ambient air 2 does not enter the first UV-germicidal irradiation device 10a.

The perforated plates 410 assist in ensuring a positive pressure inside first UV-germicidal irradiation device 10a and assist in directing the airflow through and between the perforated plates 410 and out of the first UV-germicidal irradiation device 10a.

Compared to the first UV-germicidal irradiation device 10a shown in figure 4A, the second UV-germicidal irradiation device 10b shown in figure 4B only has one interior compartment 400 with tubes 100 and light sources 200. Further, the second UV-germicidal irradiation device 10b is connected to an outlet air pipe 314 through which the exhaust air 4 exits the second UV-germicidal irradiation device 10b. The outside air 6 entering into the second air-cooling system 300b as cooling air 3 is normally directed to the second UV-germicidal irradiation device 10b by using pressure difference as explained in connection with figure 4A. The second air-cooling system 300b thus uses direct cooling of the interior compartments 400 using outside air 6 as cooling air 3 in a similar manner as the first air-cooling system 300a.

The outside air 6 entering into the second air-cooling system 300b as cooling air 3 and the exhaust air 4 exiting the second UV-germicidal irradiation device 10b, may pass through an optional heat exchange system, such as e.g. an air-to-liquid heat exchange system 302 for cooling and/or heating the outside air 6 and/or the exhaust air 4. Heating and/or cooling of the outside air 6 can be advantageous if the outside air 6 is taken directly from outside a building positioned in a climate with large temperature fluctuations and/or a climate with too low or too high temperatures compared to the temperatures optimal for cooling the light sources 200 inside the interior compartment 400. As an alternative to the air-to-liquid heat exchange system, a temperature regulating system 302 may be used. The temperature regulating system 302 typically comprises a temperature sensor configured for measuring a temperature of the outside air 6 entering into the second air-cooling system 300b, and an air regulating system.

The air regulating system is configured for:
∘ heating the air entering the one or more interior compartments 400 if the temperature of the outside air 6 measured by the temperature sensor is below a first pre-set temperature value, and
∘ cooling the air entering the one or more interior compartments 400 if the temperature of the outside air 6 measured by the temperature sensor is above a second pre-set temperature value.

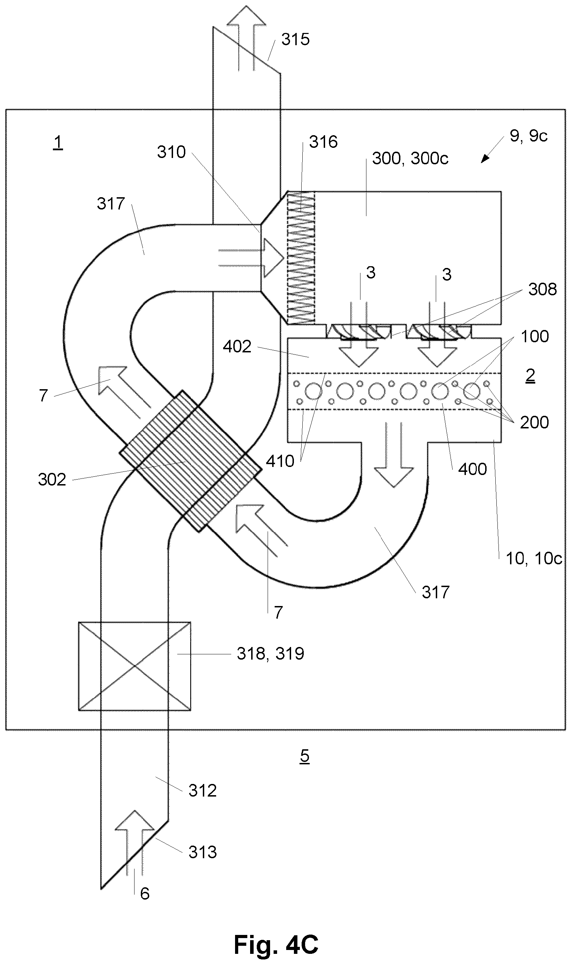

The third UV-germicidal irradiation device 10c shown in figure 4C is similar to the second UV-germicidal irradiation device 10b shown in figure 4B. Compared to the first air-cooling system 300a shown in figure 4A and the second air-cooling system 300b shown in figure 4B, the third air-cooling system 300c shown in figure 4C utilizes an internal air pipe system 317, where cooling air 3 is recycled as recycled air 7 in the internal air pipe system 317. The arrows illustrate the airflow direction. The recycled air 7 passes through a heat exchange system 302, where the recycled air 7 is cooled and/or heated by outside air 6 entering into inlet air pipe 312, which passed through the heat exchange system 302. The outside air 6 exits the inlet air pipe 312 through the outlet opening 315. Thus, the third air-cooling system 300c uses the outside air 6 for indirect cooling of the interior compartments 400. Using outside air 6 for cooling of the recycled air 7 ensures that ambient air 2 with a high salt concentration is prevented from entering into the third air-cooling system 300c and the third UV-germicidal irradiation device 10c. The heat exchange system 302 may alternatively or in addition be a valve system, which mixes the outside air 6 and the recycled air 7 before directing the mixed air to the third air-cooling system 300c.

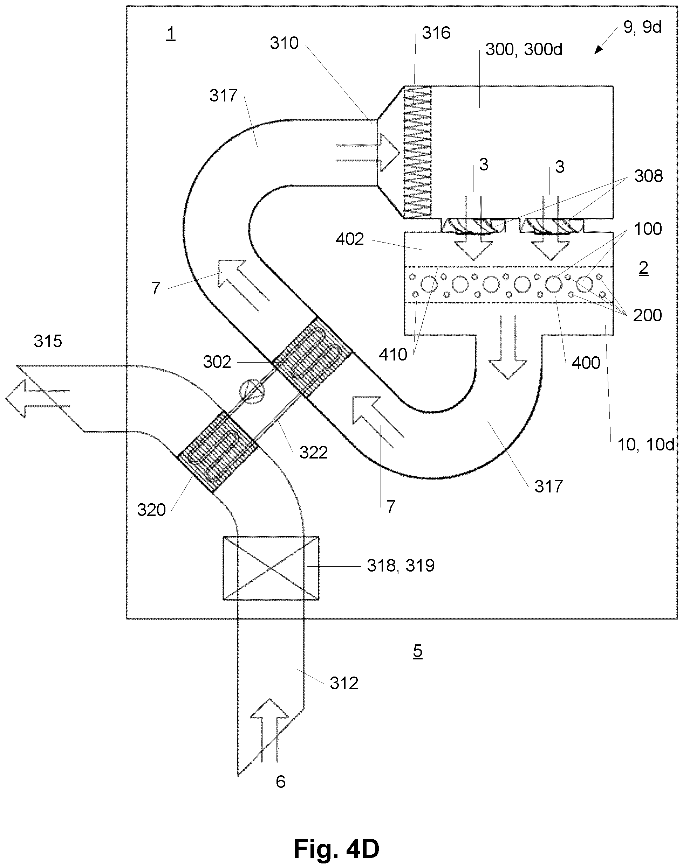

The fourth UV-germicidal irradiation device 10d shown in figure 4D is similar to the second and third UV-germicidal irradiation devices 10b, 10c shown in figures 4B and 4C. Similar to the third air-cooling system 300c shown in figure 4C, the fourth air-cooling system 300d shown in figure 4D utilizes an internal air pipe system 317, where cooling air 3 is recycled as recycled air 7 in the internal air pipe system 317. The arrows illustrate the airflow direction. The recycled air 7 passes through a heat exchange system 302 coupled to an air-to-liquid heat exchange system 320, where the recycled air 7 is cooled and/or heated by a cooling medium 322, typically water, which is again cooled by outside air 6 entering into inlet air pipe 312. The outside air 6 exits the inlet air pipe 312 through the outlet opening 315. Thus, the fourth air-cooling system 300d uses the outside air 6 for indirect cooling of the interior compartments 400. Using outside air 6 combined with the cooling medium 322 contained in a closed tubing system for cooling of the recycled air 7 ensures that ambient air 2 with a high salt concentration is prevented from entering into the fourth air-cooling system 300d and the fourth UV-germicidal irradiation device 10d. The cooling medium 8 can be water or alternatively another cooling liquid.

The invention is further described in the following items.
1. Use of a UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10), the ambient air (2) having an average salt-air concentration of at least 10 µg salt/m³ air, and
   wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for:
      A. direct cooling of the one or more light sources (200) by providing the one or more interior compartments (400) with the outside air (6) as cooling air (3), or
      B. indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7),
   wherein the outside air (6) has a salt concentration below 5 µg salt/m³ air.
2. Use of a UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10), the ambient air (2) having an average salt-air concentration of at least 10 µg salt/m³ air, and
   wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for direct cooling of the one or more light sources (200) by providing the one or more interior compartments (400) with the outside air (6) as cooling air (3), wherein the outside air (6) has a salt concentration below 5 µg salt/m³ air.
3. Use of a UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10), the ambient air (2) having an average salt-air concentration of at least 10 µg salt/m³ air, and
   wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7), wherein the outside air (6) has a salt concentration below 5 µg salt/m³ air.
4. Use of a UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10), the ambient air (2) having an average salt-air concentration of at least 10 µg salt/m³ air, and
   wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7), wherein the outside air (6) has a salt concentration lower than that of the ambient air (2) by at least 10%, such as at least 20%, such as at least 30%, such as at least 40%, such as at least 50%, such as at least 60%, such as at least 70%, such as at least 80%, such as at least 90%, such as at least 95%.
5. The use of the UV-germicidal irradiation system (9) according to any preceding item, wherein the outside air (6) has a salt concentration below 1 µg salt/m³ air.
6. The use of the UV-germicidal irradiation system (9) according to any preceding item, wherein the one or more interior compartments (400) has an interior compartment air pressure being higher than an ambient air pressure of the ambient air (2) in the first room (1) thereby preventing ambient air (2) from entering into the one or more interior compartments (400).
7. The use of the UV-germicidal irradiation system (9) according to any preceding item, wherein the one or more interior compartments (400) forms a substantially airtight system.
8. The use of the UV-germicidal irradiation system (9) according to any preceding item, wherein the air-cooling system (300) comprises one or more fans (308) configured for transferring the cooling air (3) into the UV-germicidal irradiation device (10).
9. The use of the UV-germicidal irradiation system (9) according to item 8, wherein the one or more fans (308) are configured for rotating at an adjustable velocity.
10. The use of the UV-germicidal irradiation system (9) according to any preceding item, wherein the one or more light sources (200) comprises an adjustable light source output.
11. The use of the UV-germicidal irradiation system (9) according to any preceding item, wherein the UV-germicidal irradiation system (9) comprises a temperature sensor for measuring a temperature of the cooling air (3) entering into the UV-germicidal irradiation device (10).
12. The use of the UV-germicidal irradiation system (9) according to any preceding item, wherein the UV-germicidal irradiation device (10) further comprises one or more perforated plates (410) defining the one or more interior compartments (400).
13. The use of the UV-germicidal irradiation system (9) according item 12 depending on item 2, wherein the one or more perforated plates (410) are configured for ensuring that the interior compartment air pressure is higher than an ambient air pressure of the ambient air (2) in the first room (1).
14. The use of the UV-germicidal irradiation system (9) according any of the items 2-13 depending on item 2, wherein the UV-germicidal irradiation device (10) comprises a second interior compartment (402) with a second interior compartment air pressure, the second interior compartment (402) being positioned between the one or more interior compartments (400) containing the one or more light sources (200) and the air-cooling system (300), wherein the second interior compartment air pressure is higher than the first mentioned interior compartment air pressure.
15. The use of the UV-germicidal irradiation system (9) according to any preceding item, wherein the air-cooling system (300) comprises an inlet air pipe (312) with an inlet pipe opening (313), the inlet pipe opening (313) being configured for being positioned outside (5) the first room (1).
16. The use of the UV-germicidal irradiation system (9) according to any preceding item, wherein the air-cooling system (300) comprises one or more coarse filters (319) configured for filtering larger particles from the cooling air (3) before the cooling air (3) enters into the UV-germicidal irradiation device (10).
17. The use of the UV-germicidal irradiation system (9) according to item 16 depending on item 15, wherein the one or more coarse filters (319) are positioned along the inlet air pipe (312).
18. The use of the UV-germicidal irradiation system (9) according to any preceding item, wherein the air-cooling system (300) comprises a de-humidifier (318) configured for lowering the humidity of cooling air (3) before the cooling air (3) enters into the UV-germicidal irradiation device (10).
19. The use of the UV-germicidal irradiation system (9) according to item 18 depending on item 15, wherein the de-humidifier (318) is positioned along the inlet air pipe (312).
20. The use of the UV-germicidal irradiation system (9) according to any preceding item, wherein the UV-germicidal irradiation device (10) comprises an outlet opening (315) through which the outside air (6) exits the UV-germicidal irradiation device (10) as exhaust air (4).
21. The use of the UV-germicidal irradiation system (9) according to item 20, wherein the exhaust air (4) exits through the outlet opening (315) and into the first room (1).
22. The use of the UV-germicidal irradiation system (9) according to item 20, wherein the UV-germicidal irradiation device (10) comprises an outlet air pipe (314) with the outlet opening (315), wherein the outlet air pipe (314) to a position outside (5) the first room (1), whereby the exhaust air (4) is led to a position outside (5) the first room (1).
23. The use of the UV-germicidal irradiation system (9) according to any preceding item, wherein the air-cooling system (300) comprises one or more particle filters (316) configured for filtering fine particles from the outside air (6) and/or internally recycled air (7) before the outside air (6) and/or internally recycled air (7) enters into the UV-germicidal irradiation device (10).
24. The use of the UV-germicidal irradiation system (9) according to any preceding item, wherein the system (10) further comprises a temperature regulating system (302) comprising a temperature sensor configured for measuring a temperature of the outside air (6), and/or the recycled air (7), and/or the cooling air (3) entering into the air-cooling system (300), and an air regulating system, wherein the air regulating system is configured for:
   ∘ heating the cooling air (3) entering the one or more interior compartments (400) if the temperature of the outside air (6), and/or the recycled air (7), and/or the cooling air (3) measured by the temperature sensor is below a first pre-set temperature value;
   ∘ cooling the air entering the one or more interior compartments (400) if the temperature of the outside air (6), and/or the recycled air (7), and/or the cooling air (3) measured by the temperature sensor is above a second pre-set temperature value.
25. The use of the UV-germicidal irradiation system (9) according to any preceding item, wherein the air-cooling system (300) is connected to an air-to-liquid heat exchange system (302), the air-to-liquid heat exchange system (302) comprising one or more cooling liquid containers containing a cooling liquid, wherein the air-to-liquid heat exchange system (302) is configured for heating or cooling the outside air (6) before the outside air (6) enters into the UV-germicidal irradiation device (10).
26. The use of the UV-germicidal irradiation system (9) according to item 25 depending on item 24, wherein the temperature regulating system (302) is an air-to-liquid heat exchange system (302).
27. The use of the UV-germicidal irradiation system (9) according to item 25 or 26 depending on item 15, wherein the air-to-liquid heat exchange system (302) is positioned along the inlet air pipe (312).
28. The use of the UV-germicidal irradiation system (9) according to item 25 or 26, wherein exhaust air (4) and/or recycled air (7) is led though the air-to-liquid heat exchange system (302) thereby heating the incoming outside air (6).
29. The use of the UV-germicidal irradiation system (9) according to any preceding item, wherein the UV-germicidal irradiation system (9) comprises a top flank (15) and wherein the air-cooling system (300) is positioned as part of the top flank (15).
30. The use of the UV-germicidal irradiation system (9) according to any preceding item, wherein the UV-germicidal irradiation device (10) comprises one or more light source filters (230) positioned between the one or more light sources (200) and the fluidic pathway (104) of the at least one tube (100), wherein the one or more light source filters (230) are configured for preventing light above a wavelength of 315 nm from reaching the fluidic pathway of the at least one tube (100).
31. The use of the UV-germicidal irradiation system (9) according to any preceding item, wherein the UV-germicidal irradiation device (10) comprises a plurality of tubes (100) arranged in one or more tube sections (110), and a plurality of light sources (200) arranged in one or more light source sections (210), wherein the tube sections (110) and the light source sections (210) are alternatingly arranged in a horizontal direction (H) or a vertical direction (V).
32. The use of the UV-germicidal irradiation system (9) according to item 30, wherein the light source sections (210) are separated from the tube sections (110) by one or more UV transparent materials (30).
33. The use of the UV-germicidal irradiation system (9) according to any preceding item, wherein the fluidic pathway (104) is a coil section (104) in the form of a spiral-shaped tube.
34. The use of the UV-germicidal irradiation system (9) according to item 33 further comprising at least one elongated support structure (114), wherein the spiral-shaped tube (104) is coiled around the elongated support structure along the longitudinal axis (L) of the fluidic pathway.
35. A method for UV-germicidal irradiation treatment of brine using a UV-germicidal irradiation system (9) comprising a UV-germicidal irradiation device (10) and an air-cooling system (300),
   wherein the UV-germicidal irradiation device (10) is positioned inside a first room (1) having ambient air (2) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for direct or indirect cooling of the one or more light sources (200) inside the one or more interior compartments (400); wherein the method comprises:
      - providing brine to the UV-germicidal irradiation device (10) positioned in the first room (1) with the ambient air (2) having the average salt-air concentration of at least 10 µg salt/m³ air surrounding the UV-germicidal irradiation device (10);
      - performing UV-germicidal irradiation treatment of the brine in the UV-germicidal irradiation device (10);
      - providing outside air (6) with a salt concentration below 5 µg salt/m³ air from outside (5) the first room (1) to the air-cooling system (300); and
      - cooling the one or more light sources (200) inside the one or more interior compartments (400) directly or indirectly with the outside air (6) by means of the air-cooling system (300), wherein:
         A. direct cooling of the one or more light sources (200) is obtained by proving the outside air (6) as cooling air (3) to the one or more interior compartments (400), and
         B. indirect cooling of the one or more light sources (200) is obtained by providing to the one or more interior compartments (400) with recycled air (7) as cooling air (3) for cooling the one or more light sources (200), and cooling the recycled air (7) with the outside air (6).
36. A method for UV-germicidal irradiation treatment of brine using a UV-germicidal irradiation system (9) comprising a UV-germicidal irradiation device (10) and an air-cooling system (300),
   wherein the UV-germicidal irradiation device (10) is positioned inside a first room (1) having ambient air (2) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for direct or indirect cooling of the one or more light sources (200) inside the one or more interior compartments (400); wherein the method comprises:
      - providing brine to the UV-germicidal irradiation device (10) positioned in the first room (1) with the ambient air (2) having the average salt-air concentration of at least 10 µg salt/m³ air surrounding the UV-germicidal irradiation device (10);
      - performing UV-germicidal irradiation treatment of the brine in the UV-germicidal irradiation device (10);
      - providing outside air (6) from outside (5) the first room (1) to the air-cooling system (300), wherein the outside air (6) has a salt concentration lower than that of the ambient air (2) by at least 10%, such as at least 20%, such as at least 30%, such as at least 40%, such as at least 50%, such as at least 60%, such as at least 70%, such as at least 80%, such as at least 90%, such as at least 95%; and
      - cooling the one or more light sources (200) inside the one or more interior compartments (400) directly or indirectly with the outside air (6) by means of the air-cooling system (300), wherein:
         A. direct cooling of the one or more light sources (200) is obtained by proving the outside air (6) as cooling air (3) to the one or more interior compartments (400), and
         B. indirect cooling of the one or more light sources (200) is obtained by providing to the one or more interior compartments (400) with recycled air (7) as cooling air (3) for cooling the one or more light sources (200), and cooling the recycled air (7) with the outside air (6).
37. A method for UV-germicidal irradiation treatment of brine using a UV-germicidal irradiation system (9) comprising a UV-germicidal irradiation device (10) and an air-cooling system (300),
   wherein the UV-germicidal irradiation device (10) is positioned inside a first room (1) having ambient air (2) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for direct cooling of the one or more light sources (200) inside the one or more interior compartments (400);
   wherein the method comprises:
      - providing brine to the UV-germicidal irradiation device (10) positioned in the first room (1) with the ambient air (2) having the average salt-air concentration of at least 10 µg salt/m³ air surrounding the UV-germicidal irradiation device (10);
      - performing UV-germicidal irradiation treatment of the brine in the UV-germicidal irradiation device (10);
      - providing outside air (6) with a salt concentration below 5 µg salt/m³ air from outside (5) the first room (1) to the air-cooling system (300); and
      - cooling the one or more light sources (200) inside the one or more interior compartments (400) directly with the outside air (6) by means of the air-cooling system (300), wherein direct cooling of the one or more light sources (200) is obtained by proving the outside air (6) as cooling air (3) to the one or more interior compartments (400).
38. A method for UV-germicidal irradiation treatment of brine using a UV-germicidal irradiation system (9) comprising a UV-germicidal irradiation device (10) and an air-cooling system (300),
   wherein the UV-germicidal irradiation device (10) is positioned inside a first room (1) having ambient air (2) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for indirect cooling of the one or more light sources (200) inside the one or more interior compartments (400);
   wherein the method comprises:
      - providing brine to the UV-germicidal irradiation device (10) positioned in the first room (1) with the ambient air (2) having the average salt-air concentration of at least 10 µg salt/m³ air surrounding the UV-germicidal irradiation device (10);
      - performing UV-germicidal irradiation treatment of the brine in the UV-germicidal irradiation device (10);
      - providing outside air (6) with a salt concentration below 5 µg salt/m³ air from outside (5) the first room (1) to the air-cooling system (300); and
      - cooling the one or more light sources (200) inside the one or more interior compartments (400) indirectly with the outside air (6) by means of the air-cooling system (300), wherein indirect cooling of the one or more light sources (200) is obtained by providing to the one or more interior compartments (400) with recycled air (7) as cooling air (3) for cooling the one or more light sources (200), and cooling the recycled air (7) with the outside air (6).
39. The method according to any of the items 35-38, wherein the outside air (6) has a salt concentration below 1 µg salt/m³ air.
40. The method according to any of the items 35-39, wherein performing UV-germicidal irradiation treatment of the brine in the UV-germicidal irradiation device (10) and cooling the one or more light sources (200) inside the one or more interior compartments (400) is done simultaneously.
41. A UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for:
      A. direct cooling of the one or more light sources (200) by providing the one or more interior compartments (400) with the outside air (6) as cooling air (3), or
      B. indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7),
   wherein the outside air (6) has a salt concentration below 5 µg salt/m³ air.
42. A UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for:
      A. direct cooling of the one or more light sources (200) by providing the one or more interior compartments (400) with the outside air (6) as cooling air (3), or
      B. indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7),
   wherein the outside air (6) has a salt concentration lower than that of the ambient air (2) by at least 10%, such as at least 20%, such as at least 30%, such as at least 40%, such as at least 50%, such as at least 60%, such as at least 70%, such as at least 80%, such as at least 90%, such as at least 95%.
43. A UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for direct cooling of the one or more light sources (200) by providing the one or more interior compartments (400) with the outside air (6) as cooling air (3), wherein the outside air (6) has a salt concentration below 5 µg salt/m³ air.
44. A UV-germicidal irradiation system (9) for treatment of brine, wherein the UV-germicidal irradiation system (9) comprises a UV-germicidal irradiation device (10) and an air-cooling system (300),
   wherein the UV-germicidal irradiation device (10) is positioned inside a first room (1) having ambient air (2) surrounding the UV-germicidal irradiation device (10) with an average salt-air concentration of at least 10 µg salt/m³ air, wherein the UV-germicidal irradiation device (10) comprises:
      - at least one tube (100) having:
         ∘ an inlet section (102) comprising a tube inlet (103) through which the brine enters the tube (100);
         ∘ an outlet section (106) comprising a tube outlet (107) through which the brine exits the tube (100): and
         ∘ a fluidic pathway (104) extending along a longitudinal axis (L) from the inlet section (102) to the outlet section (106);
      - one or more light sources (200) configured to illuminate the fluidic pathway (104), wherein the one or more light sources (200) emit light with at least one wavelength within a wavelength range between 180 nm and 315 nm;
      - one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
   wherein the air-cooling system (300) is configured for utilizing outside air (6) from outside (5) the first room (1) for indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7), wherein the outside air (6) has a salt concentration below 5 µg salt/m³ air.
45. The UV-germicidal irradiation system (9) according to any of the items 41-44, wherein the outside air (6) has a salt concentration below 1 µg salt/m³ air.
46. The UV-germicidal irradiation system (9) according to any of the items 41-45, wherein the one or more interior compartments (400) has an interior compartment air pressure being higher than an ambient air pressure of the ambient air (2) in the first room (1) thereby preventing ambient air (2) from entering into the one or more interior compartments (400).
47. The UV-germicidal irradiation system (9) according to any of the items 41-45, wherein the one or more interior compartments (400) forms a substantially airtight system.
48. The UV-germicidal irradiation system (9) according to any of the items 41-47, wherein the air-cooling system (300) comprises one or more fans (308) configured for transferring the cooling air (3) into the UV-germicidal irradiation device (10).
49. The UV-germicidal irradiation system (9) according to item 48, wherein the one or more fans (308) are configured for rotating at an adjustable velocity.
50. The UV-germicidal irradiation system (9) according to any of the items 41-49, wherein the one or more light sources (200) comprises an adjustable light source output.
51. The UV-germicidal irradiation system (9) according to any of the items 41-50, wherein the UV-germicidal irradiation system (9) comprises a temperature sensor for measuring a temperature of the cooling air (3) entering into the UV-germicidal irradiation device (10).
52. The UV-germicidal irradiation system (9) according to any of the items 41-51, wherein the UV-germicidal irradiation device (10) further comprises one or more perforated plates (410) defining the one or more interior compartments (400).
53. The UV-germicidal irradiation system (9) according item 52 depending on item 45, wherein the one or more perforated plates (410) are configured for ensuring that the interior compartment air pressure is higher than an ambient air pressure of the ambient air (2) in the first room (1).
54. The UV-germicidal irradiation system (9) according any of the items 45-53 depending on item 45, wherein the UV-germicidal irradiation device (10) comprises a second interior compartment (402) with a second interior compartment air pressure, the second interior compartment (402) being positioned between the one or more interior compartments (400) containing the one or more light sources (200) and the air-cooling system (300), wherein the second interior compartment air pressure is higher than the first mentioned interior compartment air pressure.
55. The UV-germicidal irradiation system (9) according to any of the items 41-54, wherein the air-cooling system (300) comprises an inlet air pipe (312) with an inlet pipe opening (313), the inlet pipe opening (313) being configured for being positioned outside (5) the first room (1).
56. The UV-germicidal irradiation system (9) according to any of the items 41-55, wherein the air-cooling system (300) comprises one or more coarse filters (319) configured for filtering larger particles from the cooling air (3) before the cooling air (3) enters into the UV-germicidal irradiation device (10).
57. The UV-germicidal irradiation system (9) according to item 56 depending on item 55, wherein the one or more coarse filters (319) are positioned along the inlet air pipe (312).
58. The UV-germicidal irradiation system (9) according to any of the items 41-56, wherein the air-cooling system (300) comprises a de-humidifier (318) configured for lowering the humidity of cooling air (3) before the cooling air (3) enters into the UV-germicidal irradiation device (10).
59. The UV-germicidal irradiation system (9) according to item 58 depending on item 55, wherein the de-humidifier (318) is positioned along the inlet air pipe (312).
60. The UV-germicidal irradiation system (9) according to any of the items 41-59, wherein the UV-germicidal irradiation device (10) comprises an outlet opening (315) through which the outside air (6) exits the UV-germicidal irradiation device (10) as exhaust air (4).
61. The UV-germicidal irradiation system (9) according to item 60, wherein the exhaust air (4) exits through the outlet opening (315) and into the first room (1).
62. The UV-germicidal irradiation system (9) according to item 60, wherein the UV-germicidal irradiation device (10) comprises an outlet air pipe (314) with the outlet opening (315), wherein the outlet air pipe (314) to a position outside (5) the first room (1), whereby the exhaust air (4) is led to a position outside (5) the first room (1).
63. The UV-germicidal irradiation system (9) according to any of the items 41-62, wherein the air-cooling system (300) comprises one or more particle filters (316) configured for filtering fine particles from the outside air (6) and/or internally recycled air (7) before the outside air (6) and/or internally recycled air (7) enters into the UV-germicidal irradiation device (10).
64. The UV-germicidal irradiation system (9) according to any of the items 41-63, wherein the system (10) further comprises a temperature regulating system (302) comprising a temperature sensor configured for measuring a temperature of the outside air (6), and/or the recycled air (7), and/or the cooling air (3) entering into the air-cooling system (300), and an air regulating system, wherein the air regulating system is configured for:
   ∘ heating the cooling air (3) entering the one or more interior compartments (400) if the temperature of the outside air (6), and/or the recycled air (7), and/or the cooling air (3) measured by the temperature sensor is below a first pre-set temperature value;
   ∘ cooling the air entering the one or more interior compartments (400) if the temperature of the outside air (6), and/or the recycled air (7), and/or the cooling air (3) measured by the temperature sensor is above a second pre-set temperature value.
65. The UV-germicidal irradiation system (9) according to any of the items 41-64, wherein the air-cooling system (300) is connected to an air-to-liquid heat exchange system (302), the air-to-liquid heat exchange system (302) comprising one or more cooling liquid containers containing a cooling liquid, wherein the air-to-liquid heat exchange system (302) is configured for heating or cooling the outside air (6) before the outside air (6) enters into the UV-germicidal irradiation device (10).
66. The UV-germicidal irradiation system (9) according to item 65 depending on item 64, wherein the temperature regulating system (302) is an air-to-liquid heat exchange system (302).
67. The UV-germicidal irradiation system (9) according to item 65 or 66 depending on item 64, wherein the air-to-liquid heat exchange system (302) is positioned along the inlet air pipe (312).
68. The UV-germicidal irradiation system (9) according to item 65 or 66, wherein exhaust air (4) and/or recycled air (7) is led though the air-to-liquid heat exchange system (302) thereby heating the incoming outside air (6).
69. The UV-germicidal irradiation system (9) according to any of the items 41-68, wherein the UV-germicidal irradiation system (9) comprises a top flank (15) and wherein the air-cooling system (300) is positioned as part of the top flank (15).
70. The UV-germicidal irradiation system (9) according to any of the items 41-69, wherein the UV-germicidal irradiation device (10) comprises one or more light source filters (230) positioned between the one or more light sources (200) and the fluidic pathway (104) of the at least one tube (100), wherein the one or more light source filters (230) are configured for preventing light above a wavelength of 315 nm from reaching the fluidic pathway of the at least one tube (100).
71. The UV-germicidal irradiation system (9) according to any of the items 41-70, wherein the UV-germicidal irradiation device (10) comprises a plurality of tubes (100) arranged in one or more tube sections (110), and a plurality of light sources (200) arranged in one or more light source sections (210), wherein the tube sections (110) and the light source sections (210) are alternatingly arranged in a horizontal direction (H) or a vertical direction (V).
72. The UV-germicidal irradiation system (9) according to item 70, wherein the light source sections (210) are separated from the tube sections (110) by one or more UV transparent materials (30).
73. The UV-germicidal irradiation system (9) according to any of the items 41-72, wherein the fluidic pathway (104) is a coil section (104) in the form of a spiral-shaped tube.
74. The UV-germicidal irradiation system (9) according to item 72 further comprising at least one elongated support structure (114), wherein the spiral-shaped tube (104) is coiled around the elongated support structure along the longitudinal axis (L) of the fluidic pathway.

### References

- 1: first room
- 2: ambient air surrounding the UV-germicidal irradiation system
- 3: cooling air
- 4: exhaust air exiting the UV-germicidal irradiation system
- 5: outside the first room
- 6: outside air from outside the first room
- 7: recycled air being recycled in the UV-germicidal irradiation system
- 9: UV-germicidal irradiation system
- 9a: first UV-germicidal irradiation system
- 9b: second UV-germicidal irradiation system
- 9c: third UV-germicidal irradiation system
- 9d: fourth UV-germicidal irradiation system
- 10: UV-germicidal irradiation device
- 10a: first UV-germicidal irradiation device
- 10b: second UV-germicidal irradiation device
- 10c: third UV-germicidal irradiation device
- 10d: fourth UV-germicidal irradiation device
- 11: first side flank
- 12: second side flank
- 13: third side flank
- 14: fourth side flank
- 15: top flank
- 16: bottom flank
- 17: brine receiving opening
- 28: door panel
- 30: UV transparent material
- 40: brine container
- 41: tube/pipe
- 100: tube
- 102: inlet section
- 103: tube inlet
- 104: fluidic pathway
- 106: outlet section
- 107: outlet tube
- 110: tube section
- 114: support structure
- 200: light source
- 210: light source section
- 230: light source filter
- 300: air-cooling system
- 300a: first air-cooling system
- 300b: second air-cooling system
- 300c: third air-cooling system
- 300d: fourth air-cooling system
- 302: heat exchange system
- 308: fan
- 310: opening to the air-cooling system
- 312: inlet air pipe
- 313: inlet pipe opening
- 314: outlet air pipe
- 315: outlet opening
- 316: particle filter
- 317: internal air pipe system
- 318: de-humidifier
- 319: coarse filter
- 320: air-to-liquid heat exchange system
- 322: cooling medium
- 400: interior compartment
- 400a: first interior compartment
- 400b: second interior compartment
- 402: second interior compartment
- 410: plate, perforated plate

## Claims

1. Use of a UV-germicidal irradiation system (9) for treatment of brine, wherein the UV-germicidal irradiation system (9) comprises a UV-germicidal irradiation device (10) and an air-cooling system (300),
wherein the UV-germicidal irradiation device (10) is positioned inside a first room (1) having ambient air (2) surrounding the UV-germicidal irradiation device (10), the ambient air (2) having an average salt-air concentration of at least 10 µg salt/m³ air, and wherein the UV-germicidal irradiation device (10) comprises:
- at least one tube (100) having:
∘ an inlet section (102) comprising a tube inlet (103) through which the brine enters the tube (100);
∘ an outlet section (106) comprising a tube outlet (107) through which the brine exits the tube (100): and
∘ a fluidic pathway (104) extending along a longitudinal axis (L) from the inlet section (102) to the outlet section (106);
- one or more light sources (200) configured to illuminate the fluidic pathway (104), wherein the one or more light sources (200) emit light with at least one wavelength within a wavelength range between 180 nm and 315 nm;
- one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
wherein the air-cooling system (300) is configured for utilizing outside air (6) from outside (5) the first room (1) for:
A. direct cooling of the one or more light sources (200) by providing the one or more interior compartments (400) with the outside air (6) as cooling air (3), or
B. indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7),
wherein the outside air (6) has a salt concentration below 5 µg salt/m³ air.

2. The use of the UV-germicidal irradiation system (9) according to any preceding claim, wherein the one or more interior compartments (400) has an interior compartment air pressure being higher than an ambient air pressure of the ambient air (2) in the first room (1) thereby preventing ambient air (2) from entering into the one or more interior compartments (400).

3. The use of the UV-germicidal irradiation system (9) according to any preceding claim, wherein the air-cooling system (300) comprises one or more fans (308) configured for transferring the cooling air (3) into the UV-germicidal irradiation device (10).

4. The use of the UV-germicidal irradiation system (9) according to any preceding claim, wherein the UV-germicidal irradiation system (9) comprises a temperature sensor for measuring a temperature of the cooling air (3) entering into the UV-germicidal irradiation device (10).

5. The use of the UV-germicidal irradiation system (9) according to any preceding claim, wherein the UV-germicidal irradiation device (10) further comprises one or more perforated plates (410) defining the one or more interior compartments (400), wherein the one or more perforated plates (410) are configured for ensuring that the interior compartment air pressure is higher than an ambient air pressure of the ambient air (2) in the first room (1).

6. The use of the UV-germicidal irradiation system (9) according any of the claims 2-5 depending on claim 2, wherein the UV-germicidal irradiation device (10) comprises a second interior compartment (402) with a second interior compartment air pressure, the second interior compartment (402) being positioned between the one or more interior compartments (400) containing the one or more light sources (200) and the air-cooling system (300), wherein the second interior compartment air pressure is higher than the first mentioned interior compartment air pressure.

7. The use of the UV-germicidal irradiation system (9) according to any preceding claim, wherein the air-cooling system (300) comprises an inlet air pipe (312) with an inlet pipe opening (313), the inlet pipe opening (313) being configured for being positioned outside (5) the first room (1).

8. The use of the UV-germicidal irradiation system (9) according to any preceding claim, wherein the air-cooling system (300) comprises:
∘ one or more coarse filters (319) configured for filtering larger particles from the cooling air (3) before the cooling air (3) enters into the UV-germicidal irradiation device (10), and/or
∘ a de-humidifier (318) configured for lowering the humidity of cooling air (3) before the cooling air (3) enters into the UV-germicidal irradiation device (10).

9. The use of the UV-germicidal irradiation system (9) according to claim 8 depending on claim 7, wherein the one or more coarse filters (319) and/or the de-humidifier (318) are positioned along the inlet air pipe (312).

10. The use of the UV-germicidal irradiation system (9) according to any preceding claim, wherein the UV-germicidal irradiation device (10) comprises an outlet opening (315) through which the outside air (6) exits the UV-germicidal irradiation device (10) as exhaust air (4),
∘ wherein the exhaust air (4) exits through the outlet opening (315) and into the first room (1), or
∘ wherein the UV-germicidal irradiation device (10) comprises an outlet air pipe (314) with the outlet opening (315), wherein the outlet air pipe (314) to a position outside (5) the first room (1), whereby the exhaust air (4) is led to a position outside (5) the first room (1).

11. The use of the UV-germicidal irradiation system (9) according to any preceding claim, wherein the air-cooling system (300) comprises one or more particle filters (316) configured for filtering fine particles from the outside air (6) and/or internally recycled air (7) before the outside air (6) and/or internally recycled air (7) enters into the UV-germicidal irradiation device (10).

12. The use of the UV-germicidal irradiation system (9) according to any preceding claim, wherein the system (10) further comprises a temperature regulating system (302) comprising a temperature sensor configured for measuring a temperature of the outside air (6), and/or the recycled air (7), and/or the cooling air (3) entering into the air-cooling system (300), and an air regulating system, wherein the air regulating system is configured for:
∘ heating the cooling air (3) entering the one or more interior compartments (400) if the temperature of the outside air (6), and/or the recycled air (7), and/or the cooling air (3) measured by the temperature sensor is below a first pre-set temperature value;
∘ cooling the air entering the one or more interior compartments (400) if the temperature of the outside air (6), and/or the recycled air (7), and/or the cooling air (3) measured by the temperature sensor is above a second pre-set temperature value.

13. The use of the UV-germicidal irradiation system (9) according to any preceding claim, wherein the air-cooling system (300) is connected to an air-to-liquid heat exchange system (302), the air-to-liquid heat exchange system (302) comprising one or more cooling liquid containers containing a cooling liquid, wherein the air-to-liquid heat exchange system (302) is configured for heating or cooling the outside air (6) before the outside air (6) enters into the UV-germicidal irradiation device (10).

14. A method for UV-germicidal irradiation treatment of brine using a UV-germicidal irradiation system (9) comprising a UV-germicidal irradiation device (10) and an air-cooling system (300),
wherein the UV-germicidal irradiation device (10) is positioned inside a first room (1) having ambient air (2) with an average salt-air concentration of at least 10 µg salt/m³ air, wherein the UV-germicidal irradiation device (10) comprises:
- at least one tube (100) having:
∘ an inlet section (102) comprising a tube inlet (103) through which the brine enters the tube (100);
∘ an outlet section (106) comprising a tube outlet (107) through which the brine exits the tube (100): and
∘ a fluidic pathway (104) extending along a longitudinal axis (L) from the inlet section (102) to the outlet section (106);
- one or more light sources (200) configured to illuminate the fluidic pathway (104), wherein the one or more light sources (200) emit light with at least one wavelength within a wavelength range between 180 nm and 315 nm;
- one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
wherein the air-cooling system (300) is configured for direct or indirect cooling of the one or more light sources (200) inside the one or more interior compartments (400);
wherein the method comprises:
• providing brine to the UV-germicidal irradiation device (10) positioned in the first room (1) with the ambient air (2) having the average salt-air concentration of at least 10 µg salt/m³ air surrounding the UV-germicidal irradiation device (10);
• performing UV-germicidal irradiation treatment of the brine in the UV-germicidal irradiation device (10);
• providing outside air (6) with a salt concentration below 5 µg salt/m³ air from outside (5) the first room (1) to the air-cooling system (300); and
• cooling the one or more light sources (200) inside the one or more interior compartments (400) directly or indirectly with the outside air (6) by means of the air-cooling system (300), wherein:
A. direct cooling of the one or more light sources (200) is obtained by proving the outside air (6) as cooling air (3) to the one or more interior compartments (400), and
B. indirect cooling of the one or more light sources (200) is obtained by providing to the one or more interior compartments (400) with recycled air (7) as cooling air (3) for cooling the one or more light sources (200), and cooling the recycled air (7) with the outside air (6).

15. A UV-germicidal irradiation system (9) for treatment of brine, wherein the UV-germicidal irradiation system (9) comprises a UV-germicidal irradiation device (10) and an air-cooling system (300),
wherein the UV-germicidal irradiation device (10) is positioned inside a first room (1) having ambient air (2) surrounding the UV-germicidal irradiation device (10) with an average salt-air concentration of at least 10 µg salt/m³ air, wherein the UV-germicidal irradiation device (10) comprises:
- at least one tube (100) having:
∘ an inlet section (102) comprising a tube inlet (103) through which the brine enters the tube (100);
∘ an outlet section (106) comprising a tube outlet (107) through which the brine exits the tube (100): and
∘ a fluidic pathway (104) extending along a longitudinal axis (L) from the inlet section (102) to the outlet section (106);
- one or more light sources (200) configured to illuminate the fluidic pathway (104), wherein the one or more light sources (200) emit light with at least one wavelength within a wavelength range between 180 nm and 315 nm;
- one or more interior compartments (400), wherein at least the one or more light sources (200) are confined inside the one or more interior compartments (400) wherein the one or more interior compartments (400) are configured for preventing ambient air (2) from entering into the one or more interior compartments (400); and
wherein the air-cooling system (300) is configured for utilizing outside air (6) from outside (5) the first room (1) for:
A. direct cooling of the one or more light sources (200) by providing the one or more interior compartments (400) with the outside air (6) as cooling air (3), or
B. indirect cooling of the one or more light sources (200) by the air-cooling system (300) comprising an internal air pipe system (317) containing internally recycled air (7), wherein the recycled air (7) is provided to the one or more interior compartments (400) as cooling air (3) for cooling the one or more light sources (200), and wherein the outside air (6) is cooled recycled air (7),
wherein the outside air (6) has a salt concentration below 5 µg salt/m³ air.
